# EUROPEAN PATENT APPLICATION

(11) **EP 2 057 941 A1**
(43) Date of publication of application: **13.05.2009**
(21) Application number: 07120133.9
(22) Date of filing: 07.11.2007
(51) Int. Cl.: A61B 5/00, G01N 21/64

(54) **Method and device for imaging an interior of an optically turbid medium**

(71) Applicant: Koninklijke Philips Electronics N.V., 5621 BA Eindhoven (NL)
(72) Inventor: The designation of the inventor has not yet been filed
(74) Representative: van Wermeskerken, Stephanie Christine

(57) **Abstract**

The invention relates to a method and a device (30, 125) for imaging an interior of an optically turbid medium (40). Light from a light source (35) is coupled into the turbid medium (40). Detection light emanating from the turbid medium (40) as a result of coupling (5) light from the light source (35) into the turbid medium (40) is collected. A first characteristic and a second characteristic of collected detection light are measured simultaneously. Next, the ratio of a first linear combination of the measured characteristics and a second linear combination of the measured characteristics is taken based on the recognition that the noise in both linear combinations is correlated.

## Description

### FIELD OF THE INVENTION

The invention relates to a method for imaging an interior of a turbid medium comprising the following steps:
- coupling irradiation light from a light source into the turbid medium;
- collecting detection light emanating from the turbid medium at at least one collection position relative to the turbid medium, with the detection light emanating from the turbid medium as result of coupling irradiation light into the turbid medium;
- simultaneously measuring at least a first characteristic and a second characteristic of the collected detection light, the collected detection light having been collected at a single collection position.

The invention also relates to a method for imaging an interior of an optically turbid medium (hereinafter: turbid medium) comprising the following steps:
- coupling irradiation light from a light source into the turbid medium;
- collecting detection light emanating from the turbid medium at at least one collection position relative to the turbid medium, with the detection light emanating from the turbid medium as a result of coupling irradiation light into the turbid medium and with the detection light comprising a first light component and a second light component, with the first light component and the second light component being characterized by mutually substantially different wavelength ranges;
- detecting the intensities of the first light component and the second light component using a detection unit.

The invention also relates to a device for imaging an interior of a turbid medium according to the methods.

### BACKGROUND OF THE INVENTION

An embodiment of the method and device for imaging an interior of a turbid medium is known from the international patent application PCT/IB2007/052823. A turbid medium is accommodated in a receiving volume. Next, light from a light source is coupled into the receiving volume. The wavelength of light generated by the light source may be used to excite a fluorescent agent comprised in the turbid medium accommodated in the receiving volume. After coupling light from the light source into the receiving volume, detection light is coupled out of the receiving volume, with the detection light being coupled out of the receiving volume emanating from the receiving volume as a result of coupling light from the light source into the receiving volume. From the above it is clear that detection light coupled out of the receiving volume comprises a first light component and a second light component with the first light component and the second light component having mutually different wavelengths. If light from different sub light sources is coupled into the receiving volume the first light component comprises light having been generated by a first sub light source, while the second light component comprises light having been generated by a second sub light source. Based on the ratio of the intensities of the detected first light component and of the detected second light component an image of an interior of the turbid medium is reconstructed. If light from the light source is used to excite a fluorescent agent comprised in the turbid medium, the first light component comprises light having been generated by the light source, while the second light component comprises light having been generated by the fluorescent agent. Now, an image is reconstructed based on the ratio of the intensities of the fluorescence light and the excitation light. It is a characteristic of the known method and device that the detection light and, hence, the first light component and the second light component are susceptible to noise which has a negative effect on the quality of a reconstructed image of an interior of the turbid medium.

### SUMMARY OF THE INVENTION

It is an object of the invention to reduce the effect of noise on the quality of a reconstructed image of an interior of the turbid medium. According to the invention this object is realized in that the method further comprises the following step:
- taking the ratio of a first linear combination of the detected characteristics and a second linear combination of the measured characteristics.

The invention is based on the recognition that part of the noise of the first characteristic and the noise of the second characteristic are correlated. Consequently, the noise of a first linear combination of the first and the second light characteristics and a second linear combination of both characteristics is also correlated and the correlated noise factors in the ratio of the first linear combination and the second linear combination will cancel. If, for instance, the first characteristic is the intensity of fluorescence light generated by a fluorescent agent comprised in the turbid medium as a result of irradiating the turbid medium with light from the light source (or a linear combination of the intensity of light from the light source and the intensity of the fluorescence light, both emanating from the turbid medium) and if the second characteristic is the intensity of light from the light source emanating from the turbid medium (or a linear combination of the intensity of light from the light source and the intensity of the fluorescence light, both emanating from the turbid medium), the correlation results from the fact that the fluorescence light is a result of irradiating the turbid medium with light from the light source and of the fact that the light from the light source in the fluorescence light at least partially share the same light path. If the output of the light source varies over time, the intensity of the fluorescence light will vary accordingly. The ratio of two linear combinations of the two characteristics, however, will be largely unaffected by the variation over time, because the correlated noise factors in the numerator and the denominator will cancel each other. A linear combination of the intensity of light from the light source and the intensity of fluorescence light may result from an imperfect filter for filtering fluorescence light from the light emanating from the turbid medium. An imperfect filter will not only let through fluorescence light, but also some light generated by the light source (see also patent application PH008928 (internal reference)).

An embodiment of the method according to the invention, wherein:
- the detection light comprises at least a first light component and a second light component characterized by mutually substantially different wavelength ranges;
- the first characteristic is a characteristic of the first light component;
- the method comprises the additional step of making at least a part of the first light component separately available from the collected collection light using a first component separator prior to the step of measuring.

This embodiment has the advantage that it covers the situation in which the first characteristic is the intensity of at least a part of the fluorescence light emanating from the turbid medium as described above. The second light component then comprises the light from the light source emanating from the turbid medium or a linear combination of the light from the light source and fluorescence light not comprised in the first component, the light from the light source and the fluorescence light both emanating from the turbid medium. The first light component may be made separately available using, for instance, an optical filter. If the second light component comprises at least two further components, such as light generated by the light source and fluorescence light not comprised in the first component, one or more of the further components may be made separately available using a second component separator. The first component separator and the second component separator may be comprised in a single component separator, such as an optical filter, dichroic mirror, grating, and prism.

The object of the invention is also realized with a method comprising the following steps:
- coupling irradiation light from a light source into the turbid medium;
- collecting detection light emanating from the turbid medium at at least one collection position relative to the turbid medium, with the detection light emanating from the turbid medium as a result of coupling irradiation light into the turbid medium and with the detection light comprising a first light component and a second light component;
- making the first light component and the second light component separately available from detection light collected at a single collection position using a component separator;
- simultaneously detecting the intensities of the first light component and the second light component using a detection unit;
- taking the ratio of a first linear combination of the detected intensities of the first light component and the second light component and a second linear combination of the detected intensities of the first light component and the second light component.

This method is a preferred embodiment of the method according to the invention described above. A further embodiment of the method according to the invention, wherein the first light component comprises excitation light having a wavelength chosen for exciting a fluorescent agent comprised in the turbid medium and wherein the second light component comprises fluorescence light generated by the fluorescent agent as a result of exciting the fluorescent agent with excitation light. This embodiment has the advantage that it reduces the effect of noise on the quality of a reconstructed image of an interior of the turbid medium, with the reconstructed image being based on the detected fluorescence light.

The invention is also a realized with a device for imaging an interior of a turbid medium comprising:
- a light source for generating light to be coupled into the turbid medium;
- a collection position for collecting detection light emanating from the turbid medium as a result of coupling light from the light source into the turbid medium;
- a measurement unit for simultaneously measuring at least a first characteristic and a second characteristic of collected detection light;
- a processing unit for taking the ratio of a first linear combination of the measured characteristics and a second linear combination of the detected characteristics.

The invention is also realized with a device for imaging an interior of a turbid medium comprising:
- a light source for generating light to be coupled into the turbid medium;
- a collection position for collecting detection light emanating from the turbid medium as a result of coupling light from the light source into the turbid medium;
- a component separator for making separately available according to wavelength a first light component and a second light component, with the first light component and the second light component initially being comprised in detection light collected at a single collection position relative to the turbid medium and being characterized by mutually different wavelength ranges;
- a detection unit for detecting the intensities of the first light component and the second light component;
- a processing unit for taking the ratio of a first linear combination of the detected intensities of the first light component and the second light component and a second linear combination of the detected intensities of the first light component and the second light component.

A further embodiment of the device according to the invention, wherein the device is a medical image acquisition device for imaging an interior of a turbid medium. This embodiment has the advantage that it enables a device for imaging an interior of a turbid medium according to the invention having a medical application.

A further embodiment of the device according to the invention, wherein the component separator comprises at least one of the elements comprised in the group comprising: a dichroic mirror, a transmission grating, and a prism. This embodiment has the advantage that a dichroic mirror, a transmission grating, and a prism are examples of component separators that can be easily implemented for separating light components characterized by mutually different wavelength ranges according to wavelength.

### BRIEF DESCRIPTION OF THE DRAWINGS

Fig. 1 schematically shows an embodiment of a method according to the invention;
Fig. 2 schematically shows an embodiment of a device for imaging an interior of a turbid medium according to the invention;
Fig. 3 schematically shows an embodiment of a medical image acquisition device for imaging an interior of a turbid medium according to the invention.

### DETAILED DESCRIPTION OF EMBODIMENTS

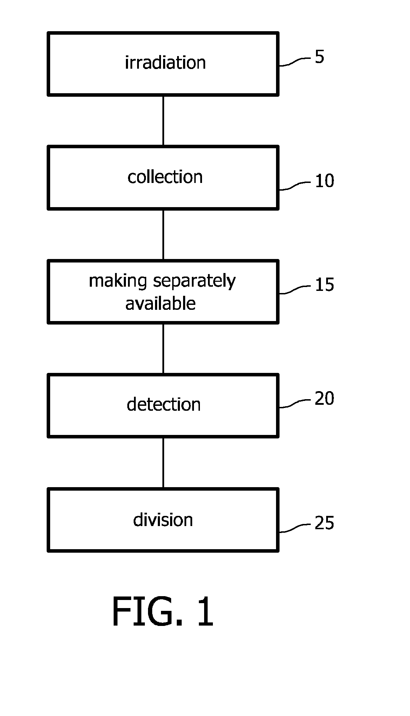

Fig. 1 schematically shows an embodiment of a method according to the invention. First, in step 5, irradiation light from a light source is coupled into the turbid medium. The irradiation light comprises a first light component and a second light component, with the different components being generated by different sub light sources comprised in the light source, and with the components being characterized by mutually different wavelength ranges. At least a part of the first light component and at least a part of the second light component then travel through the turbid medium after which they emanate from the turbid medium. Alternatively, the irradiation light is chosen such that it excites a fluorescent agent comprised in the turbid medium. In the latter case light emanating from the turbid medium comprises a first light component comprising the light from the light source that has not interacted with the fluorescent agent and a second light component comprising fluorescence light generated by the fluorescent agent in response to excitation by the irradiation light. Consequently, in both cases light emanating from the turbid medium comprises a first light component and a second light component. Light emanating from the turbid medium is called detection light. Detection light is collected at at least one collection position relative to the turbid medium. This is done in step 10. In step 15 the first light component and the second light component are made separately available from detection light collected at a single collection position according to wavelength using a component separator. Next, in step 20, the intensities of the first light component and the second light component are detected using a photo detector unit. After that, the ratio of a first linear combination of the detected intensities of the first light component and the second light component and a second linear combination of the detected intensities is taken using a processing unit. This is done in step 25. Subsequently, this ratio can be used in an image reconstruction process for reconstructing an image of an interior of the turbid medium.

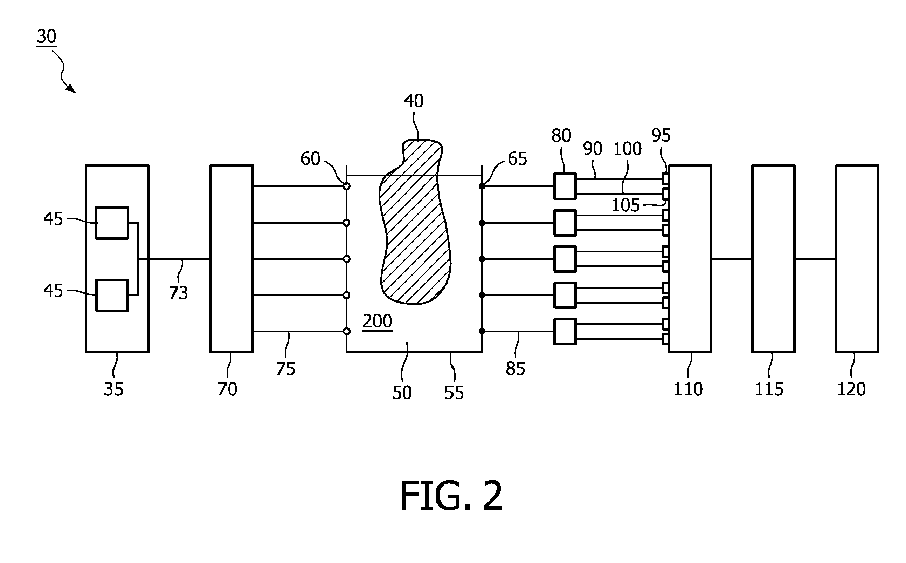

Fig. 2 schematically shows an embodiment of a device for imaging an interior of a turbid medium according to the invention. The device 30 comprises a light source 35 for generating light to be coupled into the turbid medium 40. The light source 35 may comprise a plurality of sub light sources 45, with each different sub light source generating light having a wavelength that is different from the light generated by each of the other sub light sources. The turbid medium 40 is accommodated in a receiving volume 50, which is bounded by a receptacle 55. The receptacle 55 comprises a plurality of entrance positions for light 60 for coupling light from the light source 35 into the receiving volume 50. The receptacle 55 further comprises a plurality of collection positions for light 65 for collecting light emanating from the turbid medium of 40 and, hence, from the receiving volume 50. The light source 35 is successively coupled to at least one entrance position for light chosen from the plurality of entrance positions for light 60 using the optical switch 70. The light source 35 is optically coupled to the optical switch 70 using light guide 73. The optical switch 70 is optically coupled to the receptacle 55 using light guides 75. The light source 35 may be coupled to multiple entrance positions simultaneously in that different sub light sources can be coupled to different entrance positions simultaneously. Detection light collected at a single collection position comprises a first light component and a second light component, with the components being characterized by mutually different wavelength ranges. Clearly, the detection light may comprise more than two light components, any number of which (equal to or larger than two) may be made available separately according to the invention. The different components may be the result of using a plurality of sub light sources. Alternatively, they may be the result of using light generated by the light source 35 to excite a fluorescent agent comprised in the turbid medium 40. Light generated by the light source 35 that has not interacted with the fluorescent agent is then comprised in the first light component and fluorescence light generated by the fluorescent agent in response to excitation by the light generated by the light source 35 is then comprised in the second light component (see the discussion with reference to fig.1). The first light component and the second light component comprised in detection light collected at a single collection position are made separately available from the detection light according to wavelength using a plurality of component separators 80. A dichroic mirror, a transmission grating, and a prism are examples of elements that can be used as a component separator. Detection light is coupled from the plurality of collection positions for light 65 comprised in the receptacle 55 to the component separators 80 using light guides 85. The intensities of the first light components (different first light components being collected at different collection positions for light), now communicated by the light guides 90, are detected using the plurality of detector elements 95. Similarly, the intensities of the second light components (different second light components being collected at different collection positions for light), now communicated by the light guides 100, are detected using the plurality of detector elements 105. Detector elements 95 and detector elements 105 may be comprised in a single detector unit 110. After detection of the intensities of the first light component and the second light component, the ratio of a first linear combination of the detected intensities and a second linear combination of the detected intensities is taken using processing unit 115. This ratio is then used for reconstructing an image of an interior of the turbid medium 40.

The image reconstruction process is carried out using image reconstruction unit 120. The space inside the receptacle 55 not occupied by the turbid medium 40 may be filled with a matching medium 200 having optical properties, such as absorption and scattering coefficients, that substantially match the corresponding characteristics of the turbid medium 40. In this way, boundary effects stemming from coupling light into and out of the turbid medium 40 are reduced. Moreover, use of a matching medium prevents the occurrence of an optical short-circuit. An optical short-circuit occurs if light collected at a collection position comprises both light that has passed through the turbid medium 40 and light that has passed through the receiving volume 50 but not through the turbid medium 40. As a turbid medium strongly attenuates light that passes through it, light reaching a collection position without having passed through the turbid medium 40 may dwarf the intensity of light that has passed through the turbid medium 40. This would hamper a proper measurement. When using a matching medium 200, the turbid medium 40 and the matching medium 200 effectively form a single turbid medium filling the receiving volume 50.

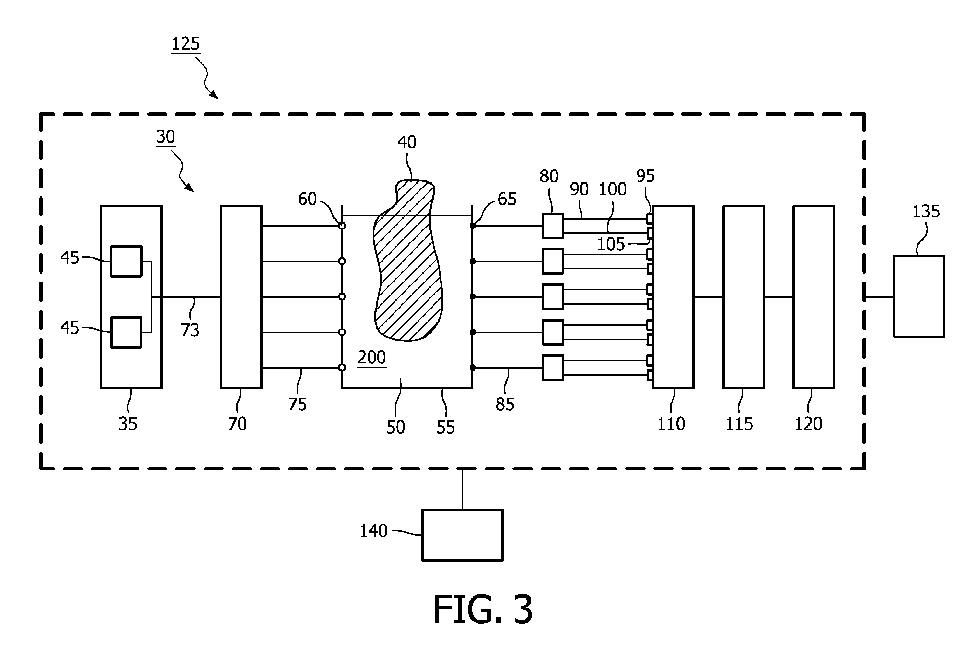

Fig. 3 schematically shows an embodiment of a medical image acquisition device for imaging an interior of a turbid medium according to the invention. The medical image acquisition device 125 comprises the elements comprised in the imaging device 30 discussed in relation to fig. 2. This is indicated by the dashed rectangle 130. The medical image acquisition device 125 further comprises a screen 135 for displaying a reconstructed image of an interior of the turbid medium 40 and an operator interface 140, for instance a keyboard, allowing an operator to interact with the medical image acquisition device 125.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements, and by means of a suitably programmed computer. In the system claims enumerating several means, several of these means can be embodied by one and the same item of computer readable software or hardware. The mere fact that certain measures are recited in mutually different dependent claims does not indicate that a combination of these measures cannot be used to advantage.

## Claims

1. A method for imaging an interior of a turbid medium (40) comprising the following steps:
- coupling (5) irradiation light from a light source (35) into the turbid medium (40);
- collecting (10) detection light emanating from the turbid medium (40) at at least one collection position (65) relative to the turbid medium (40), with the detection light emanating from the turbid medium (40) as result of coupling (5) irradiation light into the turbid medium (40);
- simultaneously measuring at least a first characteristic and a second characteristic of the collected detection light, the collected detection light having been collected at a single collection position (65);
- taking the ratio (25) of a first linear combination of the measured characteristics and a second linear combination of the measured characteristics.

2. A method as claimed in claim 1, wherein:
- the detection light comprises at least a first light component and a second light component **characterized by** mutually substantially different wavelength ranges;
- the first characteristic is a characteristic of the first light component;
- the method comprises the additional step of making at least a part of the first light component separately available from the collected collection light using a first component separator prior to the step of measuring.

3. A method for imaging an interior of a turbid medium (40) comprising the following steps:
- coupling (5) irradiation light from a light source (35) into the turbid medium (40);
- collecting (10) detection light emanating from the turbid medium (40) at at least one collection position (65) relative to the turbid medium (40), with the detection light emanating from the turbid medium (40) as a result of coupling (5) irradiation light into the turbid medium (40) and with the detection light comprising a first light component and a second light component;
- making the first light component and the second light component separately available (15) from detection light collected at a single collection position (65) using a component separator (80);
- simultaneously detecting (20) the intensities of the first light component and the second light component using a detection unit (110);
- taking the ratio (25) of a first linear combination of the detected intensities of the first light component and the second light component and a second linear combination of the detected intensities of the first light component and the second light component.

4. A method as claimed in claim 3, wherein the first light component comprises excitation light having a wavelength chosen for exciting a fluorescent agent comprised in the turbid medium (40) and wherein the second light component comprises fluorescence light generated by the fluorescent agent as a result of exciting the fluorescent agent with excitation light.

5. A device (30, 125) for imaging an interior of a turbid medium (40) comprising:
- a light source (35) for generating light to be coupled into the turbid medium (40);
- a collection position (65) for collecting detection light emanating from the turbid medium (40) as a result of coupling (5) light from the light source (35) into the turbid medium (40);
- a measurement unit (110) for simultaneously measuring at least a first characteristic and a second characteristic of collected detection light;
- a processing unit (115) for taking the ratio of a first linear combination of the measured characteristics and a second linear combination of the detected characteristics.

6. A device (30, 125) for imaging an interior of a turbid medium (40) comprising:
- a light source (35) for generating light to be coupled into the turbid medium (40);
- a collection position for collecting detection light emanating from the turbid medium (40) as a result of coupling (5) light from the light source (35) into the turbid medium (40);
- a component separator (80) for making separately available (15) a first light component and a second light component, with the first light component and the second light component initially being comprised in detection light collected at the single collection position (65) relative to the turbid medium (40);
- a detection unit (110) for detecting (20) the intensities of the first light component and the second light component;
- a processing unit (115) for taking the ratio (25) of a first linear combination of the detected intensities of the first light component and the second light component and a second linear combination of the detected intensities of the first light component and the second light component .

7. A device (30, 125) as claimed in claim 6, wherein the device (30, 125) is a medical image acquisition device (125) for imaging an interior of a turbid medium (40).

8. A device (30, 125) as claimed in claims 6-7, wherein the first light component and the second light component are **characterized by** mutually different wavelength ranges and wherein the component separator (80) is arranged for making the first light component and the second light component separately available according to wavelength.

9. A device (30, 125) as claimed in claims 6-8, wherein the component separator (80) comprises at least one of the elements comprised in the group comprising: a dichroic mirror, a transmission grating, and a prism.
